# DEMANDE DE BREVET EUROPEEN

(11) **EP 3 567 348 A1**
(43) Date de publication de la demande: **13.11.2019**
(21) Numéro de dépôt: 18305576.3
(22) Date de dépôt: 09.05.2018
(51) Int. Cl.: G01F 11/08, A61L 9/015, B01L 3/02

(54) **RESERVOIR POUR FLUIDE AMELIORE**

(71) Demandeur: B.T.H., 75001 Paris (FR)
(72) Inventeur: THITO, Brigitte, 1060 BRUXELLES (BE)
(74) Mandataire: Gauchet, Fabien Roland

(57) **Abrégé**

Le réservoir (1) pour un fluide comporte un compartiment principal (30) contenant une quantité de fluide (3), un orifice de distribution du fluide (5) en connexion fluidique avec le compartiment principal, une membrane (4) flexible déformable élastiquement agencée de sorte à former des moyens de pompage permettant une libération d'une goutte calibrée de fluide par l'orifice de distribution, un compartiment secondaire (40) positionné entre la membrane flexible et le compartiment principal, la membrane flexible délimitant en partie le compartiment secondaire et le compartiment secondaire étant en communication fluidique avec le compartiment principal via des moyens de connexion fluidique (61) au moins étanches au fluide lors d'une manipulation du réservoir.

## Description

### DOMAINE TECHNIQUE DE L'INVENTION

L'invention concerne un réservoir pour fluide, notamment un fluide aromatique, tel que des huiles essentielles ou des huiles végétales utilisées en aromathérapie.

### ETAT DE LA TECHNIQUE ANTERIEURE

Un certain nombre de fluides tels que les fluides aromatiques comme les huiles essentielles par exemple ont la particularité de se dégrader dans le temps en l'absence de précaution particulière prise pour leur conservation. Le document WO90/03192 décrit de petits réservoirs destinés à contenir et conserver de tels fluides aromatiques et qui sont de forme cylindrique comportant, en partie basse, un orifice de distribution d'une goutte calibrée de fluide aromatique et, au niveau de leur extrémité supérieure, un capuchon en élastomère flexible, déformable élastiquement de sorte à former un dispositif de type compte-goutte. Toutefois, de tels réservoirs ont l'inconvénient que, lors de la manipulation des réservoirs avant leur mise en place dans un carquois d'un distributeur, le fluide aromatique contenu vient au contact plus ou moins prolongé avec le capuchon en élastomère, ce qui augmente les risques de dégradation supplémentaire du fluide aromatique avant son utilisation dans le distributeur.

### EXPOSE DE L'INVENTION

Un but de l'invention est de fournir un réservoir qui permette d'éviter une dégradation d'un fluide qu'il contient.

A cette fin, il est prévu, selon l'invention, un réservoir pour un fluide comportant un compartiment principal contenant une quantité de fluide, un orifice de distribution du fluide en connexion fluidique avec le compartiment principal, une membrane flexible déformable élastiquement agencée de sorte à former des moyens de pompage permettant une libération d'une goutte calibrée de fluide par l'orifice de distribution, caractérisé en ce que le réservoir comporte en outre un compartiment secondaire positionné entre la membrane flexible et le compartiment principal, la membrane flexible délimitant en partie le compartiment secondaire et le compartiment secondaire étant en communication fluidique avec le compartiment principal via des moyens de connexion fluidique au moins étanches au fluide lors d'une manipulation du réservoir.

Avantageusement, mais facultativement, le réservoir selon l'invention présente au moins l'une des caractéristiques techniques suivantes:
- les compartiments principal et secondaire sont séparés par une paroi de séparation du réservoir ;
- la paroi de séparation est plane ;
- la paroi de séparation est de forme générale tronconique s'étendant dans le compartiment principal ;
- les moyens de connexion fluidique comprennent un orifice calibré ;
- l'orifice calibré est aménagé à travers la paroi de séparation ;
- l'orifice de distribution est agencé de sorte à former la goutte calibrée de fluide ;
- l'orifice de distribution est incliné par rapport à un axe longitudinal du réservoir ;
- le fluide contenu dans le compartiment principal est l'un parmi une huile essentielle et une huile végétale ; et,
- le réservoir est réalisé en partie en verre.

Il est aussi prévu, selon l'invention, un carrousel de fluides comportant une structure de réception et de support d'une pluralité de réservoirs présentant au moins l'une des caractéristiques techniques précédentes.

Il est aussi prévu, selon l'invention, un dispositif de préparation d'un mélange de fluides comportant des moyens de réception d'un carrousel présentant la caractéristique technique précédente ou d'une série de réservoirs présentant au moins l'une des caractéristiques techniques précédentes.

Il est aussi prévu, selon l'invention, un dispositif permettant la propagation de fluides comportant des moyens de réception d'un carrousel présentant la caractéristique technique précédente ou d'une série de réservoirs présentant au moins l'une des caractéristiques techniques précédentes.

Avantageusement, mais facultativement, le dispositif selon l'invention présente au moins l'une des caractéristiques techniques suivantes :
- Le dispositif comporte au moins un moyen d'actionnement agencé de sorte à venir appuyer sur la membrane flexible lors d'une distribution de la goutte calibrée de fluide ; et,
- le moyen d'actionnement comporte un électroaimant.

### BREVE DESCRIPTION DES FIGURES

D'autres caractéristiques et avantages de l'invention ressortiront à la lecture de la description qui suit d'un mode de réalisation de l'invention. Aux dessins annexés :
- la figure 1 est une vue en coupe longitudinale d'un réservoir selon un premier mode de réalisation de l'invention ;
- la figure 2 est une vue en coupe longitudinale d'un deuxième mode de réalisation d'un réservoir selon l'invention en position renversée ;
- les figures 3a à 3c sont des vues en coupes longitudinale du réservoir de la figure 2 illustrant une utilisation dudit réservoir dans un dispositif de préparation d'un mélange ou de propagation selon l'invention.

Pour plus de clarté, les éléments identiques ou similaires sont repérés par des signes de référence identiques sur l'ensemble des figures.

### DESCRIPTION DETAILLEE D'UN MODE DE REALISATION

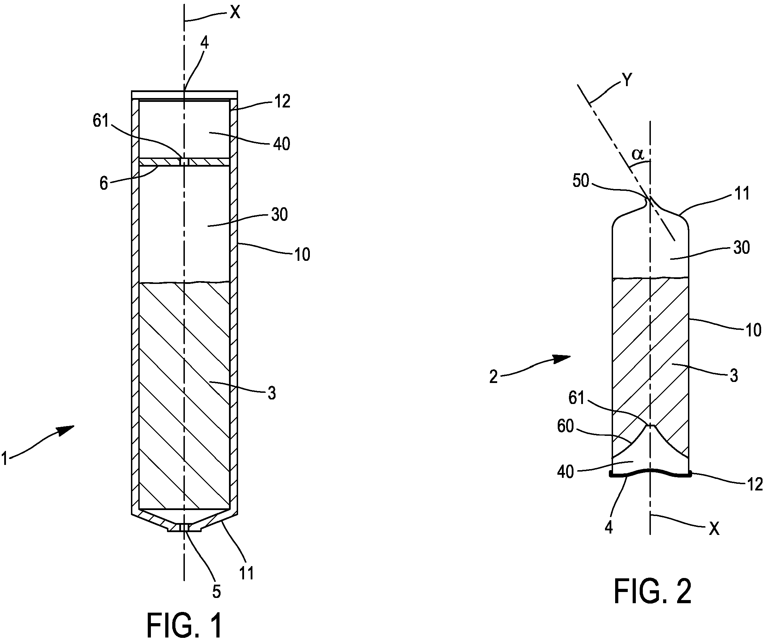

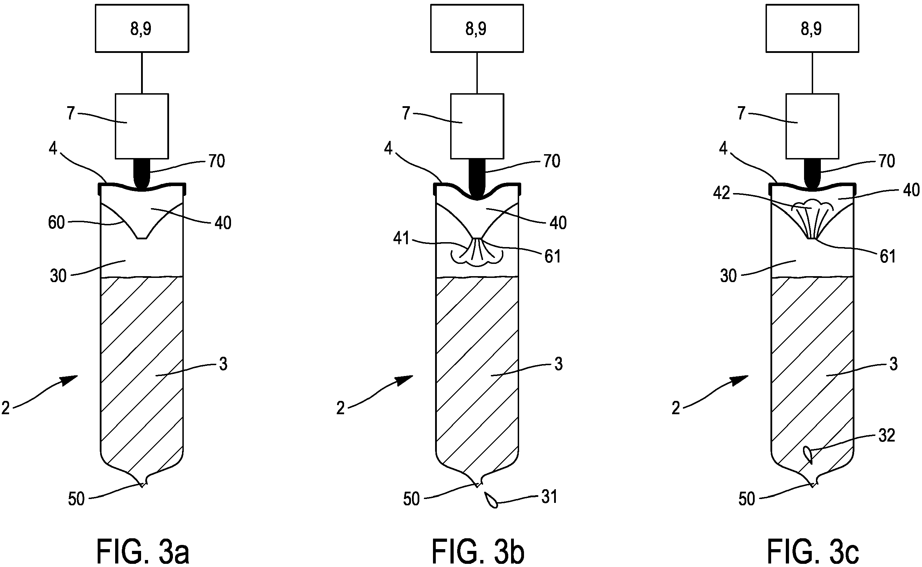

En référence à la figure 1, nous allons décrire plus en détail un premier mode de réalisation d'un réservoir pour un fluide 1 selon l'invention. Le réservoir pour un fluide 1 selon l'invention contient, ou est destiné à contenir, un fluide 3, comme par exemple, sans que cela soit limitatif, une huile essentielle ou une huile végétale, ou encore un fluide cosmétique, thérapeutique ou alimentaire. Le réservoir pour un fluide 1 selon l'invention est globalement de forme cylindrique et allongé selon un axe longitudinal X. Ici, le réservoir pour un fluide 1 selon l'invention est de forme cylindrique de révolution d'axe, l'axe longitudinal X. Il comporte une extrémité inférieure 11, selon la figure 1, et une extrémité supérieure 12. Au niveau de son extrémité inférieure 11, le réservoir pour un fluide 1 selon l'invention comporte un orifice de distribution 5 agencé de sorte que, lors d'une utilisation du réservoir pour un fluide 1 selon l'invention, il délivre une goutte calibrée 31 (voir figure 3b) de fluide 3. Ici, l'orifice de distribution 5 est orienté parallèlement à l'axe longitudinal X. De plus, il est ici coaxial audit axe longitudinal X.

S'étendant depuis l'extrémité inférieure 11 le long de l'axe longitudinal X en direction de l'extrémité supérieur 12, le réservoir pour un fluide 1 selon l'invention comporte un premier compartiment, ou compartiment principal 30, délimité latéralement par une paroi 10, et comprenant, ou destiné à recevoir, une quantité de fluide 3. Le compartiment principal 30 est délimité en partie supérieure par une paroi de séparation 6 s'étendant, ici, en travers du réservoir pour un fluide 1 selon l'invention. De plus le compartiment principal 30 est en connexion fluidique avec l'orifice de distribution 5.

Entre la paroi de séparation 6 et l'extrémité supérieure 12, le réservoir pour un fluide 1 selon l'invention comprend un deuxième compartiment, ou compartiment secondaire 40 délimité en partie basse par la paroi de séparation 6, et latéralement par la paroi 10. Le compartiment secondaire 40 est en communication fluidique avec le compartiment principal 30 via des moyens de connexion fluidique, ici comportant un orifice calibré 61 aménagé à travers une épaisseur de la paroi de séparation 6. Ces moyens de connexion fluidique sont au moins étanches au fluide 3, en particulier lors de toute manipulation du réservoir pour un fluide 1 selon l'invention. Cela veut dire que si le réservoir pour un fluide 1 selon l'invention est retourné, agité, secoué ou allongé, le fluide 3 contenu dans le compartiment principal 30 ne peut pas s'écouler ou fuir dans le compartiment secondaire 40 à travers les moyens de connexion fluidique, ici l'orifice calibré 61 a cet effet. Le compartiment secondaire 40 comporte de l'air ou tout autre gaz. Il est à noter que les moyens de connexion fluidique, ici l'orifice calibré 61, ne sont pas étanches à l'air, ou tout autre gaz contenu dans le compartiment secondaire 40, ce dernier pouvant s'écouler d'un compartiment à l'autre.

D'autre part, le réservoir pour un fluide 1 selon l'invention comporte, au niveau de son extrémité supérieure 12, une membrane 4 qui est flexible et déformable élastiquement. La membrane 4 forme un capuchon de fermeture du compartiment secondaire 40. La membrane 4 est, en outre, agencée de sorte à former des moyens de pompage, à la manière d'un système compte-goutte, permettant une libération d'une goutte calibrée 31 de fluide 3, comme cela sera expliqué ultérieurement en regard des figures 3a à 3c illustrant un deuxième mode de réalisation d'un réservoir pour un fluide 2 selon l'invention.

En référence maintenant à la figure 2, nous allons décrire le deuxième mode de réalisation du réservoir pour un fluide 2 selon l'invention. Il est à noter que le réservoir pour un fluide 2 selon l'invention est ici représenté retourné à 180° par rapport au premier mode de réalisation du réservoir pour un fluide 1 selon l'invention. Nous ne décrirons cependant que les différences qu'il présente avec ce premier mode de réalisation du réservoir pour un fluide 1 selon l'invention décrit précédemment.

Le réservoir pour un fluide 2 selon l'invention se distingue premièrement par le fait que l'orifice de distribution 50 présente un axe longitudinal Y qui est incliné d'un angle α avec l'axe longitudinal X du réservoir pour un fluide 2 selon l'invention. cela permet une libération déviée de la goutte calibrée 31 de fluide 3 lors d'une utilisation du réservoir pour un fluide 2 selon l'invention.

Ensuite, le réservoir pour un fluide 2 selon l'invention se différencie par le fait que la paroi de séparation 60 est de forme tronconique, l'orifice calibré 61 étant, par exemple, aménagé au niveau d'un sommet de la forme tronconique. En variante, l'orifice calibré 61 est réalisé au niveau d'une paroi tronconique de la paroi de séparation 60. D'autre part, la forme tronconique de la paroi de séparation 60 s'étend, ici dans le compartiment principal 30.

En variante de réalisation, le réservoir pour un fluide selon l'invention ne présente que l'une des deux différences précédentes avec le premier mode de réalisation du réservoir pour un fluide 1 selon l'invention.

L'ensemble du réservoir pour un fluide 1,2 selon l'invention est réalisé, par exemple, en verre, de préférence opaque, en dehors de la membrane 4 qui est en élastomère.

Nous allons maintenant décrire, en référence aux figures 3a à 3c, une utilisation du réservoir pour un fluide 1, 2 selon l'invention précédemment décrit au sein d'un dispositif de préparation 8 d'un mélange de fluides s selon l'invention ou d'un dispositif permettant la propagation 9 de fluides s selon l'invention.

Le distributeur 8,9 comporte un réceptacle d'un ou plusieurs réservoirs pour un fluide 1,2 selon l'invention décrits précédemment. Il est à noter qu'un carrousel de fluide selon l'invention peut être utilisé dans le réceptacle. Ce carrousel comporte à cette fin une structure de réception et de support d'une pluralité de réservoirs pour un fluide 1,2 selon l'invention, à la manière d'un carquois. Le distributeur 8,9 comporte au moins un moyen d'actionnement 7 agencé de sorte à venir appuyer sur la membrane 4 lors d'une distribution de la goutte calibrée 31. Ici le moyen d'actionnement est un électroaimant 7 manoeuvrant un doigt d'appui 70 en contact avec la membrane 4. Le distributeur peut comporter autant de moyens d'actionnement 7 qu'il y a de réservoir pour un fluide 1,2 selon l'invention au sein du dispositif 8,9.

la figure 3a illustre une position au repos entre le moyen d'actionnement 7 et le réservoir pour un fluide 1,2 selon l'invention. Dans cette position, ici, le doigt 70 est au contact sans appuyer sur la membrane 4 qui est dans une position de repos. En variante de réalisation, le doigt 70 n'est pas en contact avec la membrane et présente une extrémité libre positionnée à distance et en regard de ladite membrane 4.

Ensuite, en figure 3b, lors d'une distribution de la goutte calibrée 31, le doigt 70 appuie sur la membrane 4 en la déformant vers l'intérieur du compartiment secondaire 40, créant au sein de ce dernier une augmentation de la pression interne de l'air présent. En conséquence, un flux d'air sous pression 41 passe à travers l'orifice calibré 61 du compartiment secondaire 40 vers le compartiment principal 30, créant une surpression au sein de celui-ci. Cette surpression ainsi créée au sein du compartiment principal 30 provoque la distribution de la goutte calibrée 31 de fluide par écoulement du fluide 3 à travers l'orifice de distribution 5,50 depuis le compartiment principal 30.

Ensuite, en figure 3C, le doigt 70 relâche son appui sur la membrane 4 qui, du fait de son élasticité revient en position de repos, créant une dépression au sein du compartiment secondaire 40 qui crée à son tour un flux d'air 42 à travers l'orifice calibré 61 depuis le compartiment principal 30 vers le compartiment secondaire. Une dépression apparait alors au sein du compartiment principal 30 qui a pour conséquence l'introduction d'un bulle d'air 32 via l'orifice de distribution 5,50 au sein du compartiment principal 30.

Il ressort de ce qui précède, qu'une telle structure du réservoir pour un fluide 1,2 selon l'invention évite tout contact entre le fluide 3 et la membrane 4. Une telle situation ainsi évitée est illustrée par exemple à la figure 2 où, le réservoir pour un fluide 2 selon l'invention est complètement retourné, le fluide 3 reste à l'intérieur du compartiment principal 30 sans passer dans le compartiment secondaire 40 et ainsi le fluide 3 ne vient pas en contact avec la membrane 4.

Bien entendu, il est possible d'apporter à l'invention de nombreuses modifications sans pour autant sortir du cadre de celle-ci.

## Revendications

1. Réservoir (1;2) pour un fluide comportant un compartiment principal (30) contenant une quantité de fluide (3), un orifice de distribution du fluide (5;50) en connexion fluidique avec le compartiment principal, une membrane (4) flexible déformable élastiquement agencée de sorte à former des moyens de pompage permettant une libération d'une goutte calibrée de fluide (31) par l'orifice de distribution, **caractérisé en ce que** le réservoir comporte en outre un compartiment secondaire (40) positionné entre la membrane flexible et le compartiment principal, la membrane flexible délimitant en partie le compartiment secondaire et le compartiment secondaire étant en communication fluidique avec le compartiment principal via des moyens de connexion fluidique (61) au moins étanches au fluide lors d'une manipulation du réservoir.

2. Réservoir selon la revendication 1, **caractérisé en ce que** les compartiments principal et secondaire sont séparés par une paroi de séparation (6;60) du réservoir.

3. Réservoir selon la revendication 2, **caractérisé en ce que** la paroi de séparation (6) est plane.

4. Réservoir selon la revendication 2, **caractérisé en ce que** la paroi de séparation (60) est de forme générale tronconique s'étendant dans le compartiment principal.

5. Réservoir selon l'une des revendications 1 à 4, **caractérisé en ce que** les moyens de connexion fluidique comprennent un orifice calibré (61).

6. Réservoir selon la revendication 5 et l'une des revendications 2 à 4, **caractérisé en ce que** l'orifice calibré est aménagé à travers la paroi de séparation.

7. Réservoir selon l'une des revendications 1 à 6, **caractérisé en ce que** l'orifice de distribution est agencé de sorte à former la goutte calibrée de fluide.

8. Réservoir selon l'une des revendications 1 à 7, **caractérisé en ce que** l'orifice de distribution (50) est incliné par rapport à un axe longitudinal du réservoir.

9. Réservoir selon l'une des revendications 1 à 8, **caractérisé en ce que** le fluide contenu dans le compartiment principal est l'un parmi une huile essentielle et une huile végétale.

10. Réservoir selon l'une des revendications 1 à 9, **caractérisé en ce qu'**il est réalisé en verre.

11. Carrousel de fluides, **caractérisé en ce qu'**il comporte une structure de réception et de support d'une pluralité de réservoirs selon l'une des revendications 1 à 10.

12. Dispositif de préparation d'un mélange des fluides (8) **caractérisé en ce qu'**il comporte des moyens de réception d'un carrousel selon la revendication 11 ou d'une série de réservoirs selon l'une des revendications 1 à 10.

13. Dispositif permettant la propagation de fluides (9) **caractérisé en ce qu'**il comporte des moyens de réception d'un carrousel selon la revendication 11 ou d'une série de réservoirs selon l'une des revendications 1 à 10.

14. Dispositif (8,9) selon la revendication 12 ou 13, **caractérisé en ce qu'**il comporte au moins un moyen d'actionnement (7,70) agencé de sorte à venir appuyer sur la membrane flexible lors d'une distribution de la goutte calibrée de fluide .

15. Dispositif selon la revendication 14, **caractérisé en ce que** le moyen d'actionnement comporte un électroaimant (7).
